# EUROPÄISCHE PATENTANMELDUNG

(11) **EP 0 556 547 A2**
(43) Veröffentlichungstag der Anmeldung: **25.08.1993**
(21) Anmeldenummer: 93100190.3
(22) Anmeldetag: 08.01.1993
(51) Int. Cl.: A61B 19/02, A61L 2/26, A61M 25/00, B65D 75/30

(54) **Strahlensterilisierbare Verpackungseinheit für medizinisch-technische Gebrauchsartikel**

(30) Priorität: 17.01.1992 DE 4201078
(71) Anmelder: B. Braun Melsungen AG, D-34209 Melsungen (DE)
(72) Erfinder: Brethauer, Ulrich, W-3501 Körle (DE); Veit, Lutz, W-3500 Kassel (DE)
(74) Vertreter: Jönsson, Hans-Peter, Dr.Dipl.-Chem.

(57) **Zusammenfassung**

Gegenstand der Erfindung sind strahlensterilisierbare Verpackungseinheiten für medizinisch-technische Gebrauchsartikel bestehend aus zwei trennbar miteinander kleberfrei verbundenen, jeweils coextrudierten ein Lumen bildenden Folien aus physiologisch unbedenklichem wiederverwertbaren Kunststoffmaterial, wobei die Folien außerhalb des Lumens in wenigstens einem Teilbereich der angrenzenden Flächen getrennt voneinander angeordnet sind, die transparent durchsichtige Grundfolie und die opake, wenigstens einseitig bedruckbare Deckelfolie aus einem Styrol-homo- oder -copolymerisat oder aus einem Ethylen-homo- oder -copolymerisat besteht.

## Beschreibung

Die Erfindung betrifft strahlensterilisierbare Verpackungseinheiten für medizinisch-technische Gebrauchsartikel, bestehend aus zwei trennbar miteinander kleberfrei verbundenen, ein Lumen bildenden Folien aus physiologisch unbedenklichem wiederverwertbarem Kunststoffmaterial.

Medizinisch-technische Gebrauchsartikel, beispielsweise Infusionsgeräte, werden typischerweise mit einer transparenten Polyamid/Polyethylen-Verbundfolie gesiegelt gegen ein Sterilpapier verpackt. Die Verwendung von mehr oder weniger gasdurchlässigem Papier erlaubt die Sterilisation der in der Verpackung befindlichen Gebrauchsartikel durch Einwirkung von Ethylenoxidgas. Die Sterilisation mittels Ethylenoxidgas hat jedoch verschiedene Nachteile, beispielsweise verbleiben Reste des Etylenoxids in den medizinisch-technischen Gebrauchsartikeln und werden teilweise erst bei der medizinisch-technischen Anwendung freigesetzt. Darüberhinaus genügt eine derartige zweikomponentige Anordnung (Papier und Kunststoffmaterial) nicht den jüngsten Anforderungen der Verpackungsverordnungen, da zur Wiederverwendung das Sterilpapier von der Folie getrennt werden muß. Darüberhinaus ist eine Wiederverwendung der kleberkaschierten Polyamid/Polyethylen-Verbundfolie ausgeschlossen.

Strahlensterilisationsversuche mit energiereicher Strahlung, beispielsweise Gammastrahlung, haben am Papier Abbauerscheinungen gezeigt, die unter anderem die sogenannte Peelfähigkeit mindern. Hierunter ist zu verstehen, daß Papier und Verbundfolie nur schlecht ohne Zuhilfenahme von Hilfsmitteln voneinander getrennt werden können. Dies ist jedoch von besonderer Bedeutung, beispielsweise bei der Verpackung von Infusionsgeräten, bei denen eine schnelle und einfache Öffnung der Verpackung ohne Verwendung von Hilfsmitteln wie Messer oder Scheren erforderlich ist. Darüberhinaus wurde festgestellt, daß die Durchstoßfestigkeit des Papiers im Anschluß an die Strahlensterilisation ebenfalls vermindert worden ist. Dies führt leicht zu Beschädigungen des Papiers und damit zu möglichen Beeinträchtigungen des Inhalts. Aus JP 59 030 844 ist ein Verpackungsmaterial für den medizinischen Bereich bekannt, das Polystyrol, Polystyrol-Polybutadien-Copolymere und weitere Polymere wie SBR (Styrol-Butadien-Gummi), Methylmethacrylat-Styrol-Copolymere oder Acrylnitril-Styrol-Copolymere enthalten. Darin eingepackte Katheter oder Blutbehälter können durch Strahlung sterilisiert werden. Das Verpackungsmaterial ist transparent.

Aus der GB-B-1,123,072 sind Verpackungseinheiten für medizinisch-technische Gebrauchsartikel aus zwei mittels eines Siegelmediums aus Polypropylen verbundenen, laminierten, ein Lumen bildenden Folien aus vornehmlich Mehrschichtverbundmaterialien unter Verwendung von Aluminiumfolie bekannt. Durch die Gamma-Bestrahlung mit einer Energiedosis von 25 kGy versprödet das Siegelmedium und bewirkt die Peelfähigkeit. Bei längeren Lagerzeiten der so hergestellten Verpackungseinheiten, ist jedoch eine bakteriendichte Abdichtung der Verbindung zwischen der Grundfolie und der Deckelfolie nicht zu gewährleisten.

Bekanntlich wird aber für derartige Verpackung eine Langzeitstabilität und -Sicherheit von 5 Jahren verlangt.

Demgemäß besteht die vornehmliche Aufgabe der vorliegenden Erfindung darin, eine vollständig strahlensterilisierbare Verpackungseinheit für medizinisch-technische Gebrauchsartikel zur Verfügung zu stellen, die aus zwei trennbar miteinander kleberfrei verbundenen, jeweils coextrudierten, ein Lumen bildenden Folien aus physiologisch unbedenklichem wiederverwertbaren Kunststoffmaterial besteht. Weitere Anforderungen an die genannten strahlensterilisierbaren Verpackungseinheiten sind, daß die Folien außerhalb des Lumens in wenigstens einem Teilbereich der angrenzenden Flächen getrennt voneinander angeordnet sind, wodurch es möglich ist, die Grundfolie von der Deckelfolie ohne Verwendung von Hilfsmitteln abzuziehen. Weiterhin ist es zwar erforderlich, daß die Grundfolie transparent durchsichtig ist, um eine zweifelsfreie Erkennung des darin befindlichen Gegenstandes zu ermöglichen. Jedoch sollte die Deckelfolie opak und bedruckbar sein, um eine eindeutige Kennzeichnung des Gegenstandes zu erlauben und Hinweise auf die Handhabung geben zu können.

Die Lösung der vorgenannten Aufgaben besteht erfindungsgemäß in einer strahlensterilisierbaren Verpackungseinheit für medizinisch-technische Gebrauchsartikel, bestehend aus zwei trennbar, miteinander kleberfrei verbundenen, jeweils coextrudierten, ein Lumen bildenden Folien aus physiologisch unbedenklichem wiederverwertbaren Kunststoffmaterial, wobei die Folien außerhalb des Lumens in wenigstens einem Teilbereich der angrenzenden Flächen getrennt voneinander angeordnet sind, die transparent durchsichtige Grundfolie und die opake, wenigstens einseitig bedruckbare Deckelfolie jeweils in gleicher Weise aus einem Styrol-homo- oder -copolymerisat oder aus einem Ethylen-homo-oder -copolymerisat bestehen.

Strahlensterilisierbar im Sinne der vorliegenden Erfindung bedeutet, daß die Komponenten der Verpackungseinheit gegenüber Strahlungsquellen wie Kobalt-60-Quellen bei Raumtemperatur und Strahlendosen von 15 bis 50 kGy gegenüber stabil sind. Eine gelegentlich auftretende geringe Gelbfärbung, bedingt durch die Strahlensterilisation, ist hierbei von untergeordneter Bedeutung, insbesondere wenn diese ohne scheinbare äußere Einwirkung wieder verschwindet. Zur Gewährleistung der Sterilität des Inhaltes der Verpackungseinheit ist jedoch eine Versprödung der Bestandteile der Einheit von Nachteil.

Die erfindungsgemäßen Verpackungseinheiten sind auch über den geforderten Zeitraum, beispielsweise 5 Jahre, bakteriendicht, da die Komponenten der Verpackungseinheit gegenüber der Energiedosis bei der Sterilisation stabil sind. Es tritt auch keine Versprödung auf.

In einer bevorzugten Ausführungsform der vorliegenden Erfindung bildet die Grundfolie einen thermogeformten offenen Behälter, der mit der Deckelfolie planeben an seinem Umfang verschlossen ist. Derartige offene Behälter sind beispielsweise aus dem Lebensmittelbereich, beispielsweise der Salat- und Fleischverpackung bekannt. Für die Gewährleistung der Sterilität der medizinisch-technischen Gebrauchsartikel ist von besonderer Bedeutung, daß die Verbindung zwischen der Grundfolie und der Deckelfolie bakteriendicht ist. Dementsprechend ist in einer besonders bevorzugten Ausführungsform wenigstens eine der Folien heißsiegelfähig. Diese Art der Verbindung erlaubt ein bakteriendichtes Verschweißen der ein Lumen bildenden Folien. Diese Verbindung ist auch gegenüber energiereicher Strahlung beständig, während viele Kleber gegen Gamma-Strahlung nicht beständig sind. Darüberhinaus hat die heißgesiegelte Verbindung der Grundfolie mit der Deckelfolie den Vorteil, daß die so verbundenen Folien leicht voneinander abziehbar sind, wenn entsprechende freie Flächen der Folien zur Verfügung stehen, die mit den Fingern auseinander gezogen werden können. Es werden daher erfindungsgemäß keine Hilfsmittel, wie Scheren oder Messer, benötigt, um die Verpackungseinheit zu öffnen.

Vornehmlich aus dem Lebensmittelbereich und auch der medizinisch-technischen Verpackung sind eine Reihe von Kunststoffmaterialien bekannt, die den genannten Anforderungen an die Grundfolie entsprechen. Im Sinne der vorliegenden Erfindung besteht das Kunststoffmaterial der Grundfolie aus Styrol-homo- oder -copolymerisat, insbesondere aus biaxial gerecktem Styrol und Styrol/Butadien-Blockcopolymeren.

Biaxial orientierte (gereckte) Styrole sind glasklar, hochbrillant und umweltschonend bei hoher Schlagfestigkeit. Beispielsweise werden derartige Folien zu Schalen, Becher, Blisterpackungen, Sichtfenstern und Verpackungseinsätzen thermogeformt. Standard-Polystyrol ist ebenfalls glasklar und brillant, jedoch relativ spröde, so daß es in unmodifizierter Form kaum für den Verpackungssektor in Frage kommt. Durch eine biaxiale Reckung läßt sich jedoch die Zähigkeit von Standard-Polystyrolen erheblich steigern, so daß formstabile, aber auch ausreichend zähe Verpackungselemente erhalten werden. Biaxial orientiertes Styrol läßt sich, wie alle Polystyrole, problemlos wiederverwerten. Analog können auch beispielsweise Styrol/Butadien-Blockcopolymere im Sinne der vorliegenden Erfindung eingesetzt werden. Von diesen ist bekannt, daß diese für das Spritzgießen, Extrudieren und Thermoformen, einsetzbar sind. In einer bevorzugten Ausführungsform der vorliegenden Erfindung ist das Styrol der coextrudierten Deckelfolie ausgewählt aus Styrol/Butadien-Blockcopolymeren und Polyestern, insbesonders Polyethylenterephthalaten und Polybutylenterephtalaten. Wesentlich im Sinne der vorliegenden Erfindung ist die gemeinsame Auswahl der Bestandteile der Grundfolie und der Deckelfolie, deren chemische Zusammensetzung sich von vergleichbaren Grundbausteinen ableitet um eine optimale Wiederverwertung zu ermöglichen. In gleicher Weise wie die oben genannten Styrole sind Ethylen-homo- oder -copolymerisate einsetzbar. Besonders bevorzugt im Sinne der vorliegenden Erfindung sind die Ethylenhomopolymere ausgewählt aus transparenten und tiefziehfähigen einschichtig oder mehrschichtig aufgebauten Folien aus MDPE und LDPE. Das Polyethylen mit der niedrigen Dichte (LDPE) dient hierbei vorzugsweise als Siegelmedium, insbesondere wenn eine derartige Folie mittels Coextrusion in Verbindung mit einem Polyethylen mittlerer Dichte (MDPE) co-extrudiert worden ist. Besonders bevorzugt ist der Einsatz von Ethylen-homo- oder -copolymerisaten für die Grundfolie oder Deckelfolie mit einer Dichte im Bereich von 0,88 bis 0,965 g/cm³.

Als Beispiel für eine Weich-Verpackung für Infusions- und Transfusionsgeräte auf Styrolbasis mit einer opaken Deckelfolie, die die beidseitige Bedruckung erlaubt, kann eine Folie mit folgendem Aufbau eingesetzt werden:
20 µm Polystyrolcopolymer mit sehr guter Wärmebeständigkeit in Verbindung mit Polybutylentherephtalat (Valox^{R} von General Electric),
85 µm Polystyrolträgerschicht und
15 µm einer Schicht auf Polyesterbasis (Polybutylentherephtalat) als Siegel- oder Peelschicht.
Als Beispiel für einen zweischichtigen Aufbau der Deckelfolie sei eine opake Folie aus einer 35 µm dicken Schicht aus Polyethylen mit mittlerer Dichte (MDPE) und 12 µm Polyethylen mit niedriger Dichte (LDPE) als Siegel-Peelmedium genannt.

Obwohl die Auswahl des Kunststoffmaterials der Deckelfolie prinzipiell unabhängig von der Auswahl der Kunststoffmaterialien der Grundfolie ist, besteht jedoch ein wesentliches Merkmal der vorliegenden Erfindung darin, daß das Kunststoffmaterial der Grundfolie und der Deckelfolie von denselben Monomeren abgeleitet ist. Hierunter ist zu verstehen, daß beispielsweise für den Fall, daß die Grundfolie aus im wesentlichen Styrol besteht, auch die Deckelfolie vorzugsweise aus Styrol hergestellt werden sollte. Gleiches gilt für den Fall der Herstellung der Grundfolie aus Ethylen. In diesem Fall ist die Herstellung der Deckelfolie vorzugsweise aus Polyethylen vorzusehen. Eine entsprechende Auswahl der Kunststoffmaterialien fördert die Wiederverwertung der eingesetzten Materialien. Als Material für den Aufbau einer Grundfolie hat sich beispielsweise ein Coextrudat aus 30 µm MDPE und 90 µm LDPE oder 100 µm Styrol-Butadien-Blockcopolymer (Styrolux^{R} KR 2 786) als Blasfolie sowie für eine Hartverpackung 200 µm des genannten Styrol-Butadien-Blockcopolymer in Abmischung mit Styrol als Flachfolie als vorteilhaft herausgestellt.

In einer weiteren bevorzugten Ausführungsform der vorliegenden Erfindung ist die Deckelfolie beidseitig bedruckbar.

Weiterer Gegenstand der vorliegenden Erfindung ist die Verwendung der Verpackungseinheit zum Verpacken von Medical-Produkten, insbesondere Produkte für Infusion, Transfusion, Venenpunktion, Anästhesie, Biopsie, Spritzen, Kanülen, Urinableitung, Wunddrainage, Pleurapunktion, Pleuradrainage, Peritoneallavage, Implantate, Intra- und postoperatives Absaugen, Enterale Ernährung, Sauerstofftherapie und Hygiene-Bedarf und anschließende Sterilisation des Packungsinhaltes mittels Gammastrahlung.

## Patentansprüche

1. Strahlensterilisierbare Verpackungseinheit für medizinischtechnische Gebrauchsartikel bestehend aus zwei trennbar miteinander kleberfrei verbundenen, jeweils coextrudierten, ein Lumen bildenden Folien aus physiologisch unbedenklichem wiederverwertbaren Kunststoffmaterial, wobei die Folien außerhalb des Lumens in wenigstens einem Teilbereich der angrenzenden Flächen getrennt voneinander angeordnet sind, die transparent durchsichtige Grundfolie und die opake, wenigstens einseitig bedruckbare Deckelfolie jeweils in gleicher Weise aus einem Styrol-homo- oder -copolymerisat oder aus einem Ethylen-homo- oder -copolymerisat bestehen.

2. Verpackungseinheit nach Anspruch 1, dadurch gekennzeichnet, daß die Grundfolie einen thermogeformten offen Behälter bildet, der mit der Deckelfolie planeben an seinem Umfang verschlossen ist.

3. Verpackungseinheit nach Anspruch 1 oder 2, dadurch gekennzeichnet, daß wenigstens eine der Folie heißsiegelfähige Eigenschaften aufweist.

4. Verpackungseinheit nach einem oder mehreren der Ansprüche 1 bis 3, dadurch gekennzeichnet, daß das Styrol-homo-oder -copolymerisat der Grundfolie ausgewählt ist aus biaxial gerecktem Styrol und Styrol/Butadien-Blockcopolymeren.

5. Verpackungseinheit nach einem oder mehreren der Ansprüche 1 bis 4, dadurch gekennzeichnet, daß das Styrol-homo-oder -copolymerisat der Deckelfolie ausgewählt ist aus Styrol/Butadien-Blockcopolymeren und Polyestern.

6. Verpackungseinheit nach Anspruch 5, dadurch gekennzeichnet, daß die Polyester abgeleitet sind von Polyethylenterephthalaten und Polybutylenterephthalaten.

7. Verpackungeinheit nach einem oder mehreren der Ansprüche 1 bis 6, dadurch gekennzeichnet, daß das Ethylen-homo- oder -copolymerisat der Grundfolie oder der Deckelfolie eine Dichte im Bereich von 0,88 bis 0,965 g/cm³ aufweist.

8. Verpackungseinheit nach einem oder mehreren der Ansprüch 1 bis 7, dadurch gekennzeichnet, daß die Deckelfolie beidseitig bedruckbar ist.

9. Verwendung der Verpackungseinheit nach einem oder mehreren der Ansprüche 1 bis 8, zur Verpackung von Medical-Produkten, insbesondere Produkte für Infusion, Transfusion, Venenpunktion, Anästhesie, Biopsie, Spritzen, Kanülen, Urinableitung, Wunddrainage, Pleurapunktion, Pleuradrainage, Peritoneallavage, Implantate, Intra- und postoperatives Absaugen, Enterale Ernährung, Sauerstofftherapie und Hygiene-Bedarf und anschließender Sterilisation des Packungsinhaltes mittels Gamma-Strahlung.
